# EUROPÄISCHE PATENTSCHRIFT

(11) **EP 3 626 306 B1**
(45) Veröffentlichungstag und Bekanntmachung des Hinweises auf die Patenterteilung: **30.10.2024**
(21) Anmeldenummer: 18195211.0
(22) Anmeldetag: 18.09.2018
(51) Int. Cl.: A61N 1/375, A61N 1/39, H01G 9/08, H01G 4/40

(54) **STAPELBARER KONDENSATOR FÜR EIN IMPLANTIERBARES MEDIZINISCHES GERÄT**
STACKABLE CAPACITOR FOR AN IMPLANTABLE MEDICAL DEVICE
CONDENSATEUR EMPILABLE UN APPAREIL MÉDICAL IMPLANTABLE

(43) Veröffentlichungstag der Anmeldung: 25.03.2020
(73) Patentinhaber: BIOTRONIK SE & Co. KG, 12359 Berlin (DE)
(72) Erfinder: Henschel, Martin, 13125 Berlin (DE)
(74) Vertreter: Biotronik Corporate Services SE

(56) Entgegenhaltungen:
- US-A- 5 814 082
- US-A1- 2006 061 938
- US-B1- 7 031 139

## Beschreibung

Die vorliegende Erfindung betrifft einen Kondensator, eine Kondensatoranordnung sowie ein implantierbares medizinisches Gerät, das eine erfindungsgemäße Kondensatoranordnung bzw. einen erfindungsgemäßen Kondensator aufweist.

Hinsichtlich der Anordnung von Kondensatoren in einem Gerätegehäuse eines solchen medizinischen Geräts ist es aus dem Stand der Technik bekannt, drei baugleiche quaderförmige Kondensatoren in der gleichen Orientierung übereinander zu stapeln. Alternativ hierzu werden auch zwei baugleiche Kondensatoren sowie eine gespiegelte Bauform in einem dreifachen Stapel angeordnet, um verrundete Außenradien an der Ober- und der Unterseite des Stapels zu erhalten.

Werden drei baugleiche Kondensatoren übereinander in der gleichen Orientierung gestapelt, lässt sich bei verrundeter Oberseite des jeweiligen Kondensators keine ergonomische Verrundung auf der Unterseite erzielen. Bei nicht symmetrischer Kondensatorkontur müsste man für die Unterseite einen gespiegelten Kondensator entwickeln, was den Entwicklungsaufwand und die Teilevielfalt in nachteiliger Weise erhöhen würde. Um den Entwicklungsaufwand und die Teilevielfalt zu reduzieren wurden daher bisher drei baugleiche Kondensatoren verwendet und ein weniger ergonomisches Gehäusedesign in Kauf genommen.

Weiterhin beschreibt die US 2017/0076874 A1 einen Stapel von halbrunden Folien, die einen Kondensator bilden, der einen elektrischen Anschluss an einem Eckbereich eines Gehäuses des Kondensators aufweist.

US 5,814,082 offenbart eine Kondensatoranordnung für ein implantierbares medizinisches Gerät.

Hiervon ausgehend liegt der vorliegenden Erfindung die Aufgabe zugrunde, einen Kondensator, eine Kondensatoranordnung sowie ein implantierbares medizinisches Gerät mit einer solchen Kondensatoranordnung zu schaffen, wobei der Kondensator so ausgestaltet sein soll, dass er sich in platzsparender Weise stapeln lässt und dabei gut in ein Gerätegehäuse eines implantierbaren medizinischen Gerätes integrieren lässt.

Diese Aufgabe wird durch einen Kondensatoranordnung mit den Merkmalen des Anspruchs 1und ein implantierbares medizinisches Gerät mit den Merkmalen des Anspruchs 6 gelöst. Vorteilhafte Ausführungsformen dieser Erfindungsaspekte sind in den entsprechenden Unteransprüchen angegeben und werden nachfolgend beschrieben.

Gemäß Anspruch 1 wird ein Kondensatoranordnung offenbart, welche drei baugleiche Kondensatoren aufweist. Dabei umfasst jeder der baugleichen Kondensatoren ein Gehäuse, wobei das Gehäuse eine erste Außenseite sowie eine der ersten Außenseite abgewandte zweite Außenseite aufweist, wobei die beiden Außenseiten über eine in einer Umfangsrichtung des Gehäuses umlaufende laterale Außenseite des Gehäuses miteinander verbunden sind, und wobei die laterale Außenseite einen ebenen ersten Abschnitt sowie einen bogenförmigen zweiten Abschnitt aufweist, wobei der erste Abschnitt zwei Endbereiche aufweist, die über einen mittleren Bereich des ersten Abschnitts miteinander verbunden sind, wobei die Endbereiche jeweils mit dem zweiten Abschnitt verbunden sind, und wobei der Kondensator einen auf dem mittleren Bereich des ersten Abschnitts der lateralen Außenseite angeordneten elektrischen Anschluss aufweist, der zwei Kontaktflächen zum elektrischen Kontaktieren des Kondensators aufweist. Des Weiteren bildet der zweite Abschnitt der lateralen Außenseite mit der ersten Außenseite eine abgerundete Gehäusekante des Gehäuses des Kondensators aus. Hierbei liegt der Verrundungsradius dieser Kante vorzugsweise oberhalb von 2 mm. Weiterhin Erfindungsgemäß ist insbesondere vorgesehen, dass die drei baugleichen Kondensatoren einen ersten äußeren Kondensator, einen zweiten mittleren Kondensator und einen dritten äußeren Kondensator, wobei der zweite Kondensator zwischen den beiden äußeren Kondensatoren angeordnet ist, und wobei der zweite Kondensator mit seiner zweiten Seiten an der zweiten Seite des dritten Kondensators anliegt, und wobei der zweite Kondensator mit seiner ersten Seite an einer zweiten Seite des ersten Kondensators anliegt, und der elektrische Anschluss des dritten Kondensators versetzt zu den elektrischen Anschlüssen des ersten und des zweiten Kondensators der Kondensatoranordnung angeordnet ist.

Die baugleichen Kondensatoren lassen sich mit Vorteil in einem 3er-Stapel so anordnen, dass sowohl die Oberseite des Kondensatorstapels als auch die Unterseite des Stapels Verrundungen aufweisen kann. Das umgebende Gerätegehäuse eines implantierbaren medizinischen Geräts kann dann ebenfalls mit großen Außenradien dieser Kondensatorkontur folgen. Wird der Kondensator innerhalb eines implantierbaren Geräts angeordnet, kann durch die großen Außenradien sowie besagter Vorrundung ein Außengehäuse für das implantierbare Gerät so gestaltet sein, dass sich Vorteile für den Implantatträger ergeben, da der Tragekomfort unter der Haut im Vergleich zu einem Implantatgehäuse mit geringeren Außenradien und geringeren Vorrundung erhöht ist.

Weiterhin ist gemäß einer Ausführungsform vorgesehen, dass das Gehäuse der baugleichen Kondensatoren spiegelsymmetrisch zu einer Symmetrieebene ausgebildet ist, die senkrecht zum ersten Abschnitt der lateralen Außenseite sowie senkrecht zu der ersten und der zweiten Außenseite verläuft.

Weiterhin ist gemäß einer Ausführungsform vorgesehen, dass die erste und/oder die zweite Außenseite halbkreisförmig ausgebildet ist bzw. sind.

Gemäß einer Ausführungsform liegt der Verrundungsradius verhältnismäßig im Bereich 16% bis 33% der Dicke eines entsprechenden implantierbaren Geräts. Das Verhältnis ist so gewählt, dass bei erhöhtem Tragekomfort für den Implantatträger der Raum innerhalb eines Implantats durch den Kondensator ausgefüllt ist.

Weiterhin ist gemäß einer Ausführungsform vorgesehen, dass der erste Abschnitt der lateralen Außenseite in der Umfangsrichtung einen Mittelpunkt aufweist, und dass der elektrische Anschluss in der Umfangsrichtung einen Mittelpunkt aufweist, wobei der Mittelpunkt des elektrischen Anschlusses in der Umfangsrichtung zum Mittelpunkt des ersten Abschnitts versetzt angeordnet ist, so dass insbesondere die elektrischen Anschlüsse zweier erfindungsgemäßer Kondensatoren in der Umfangsrichtung versetzt zueinander angeordnet sind, wenn die zweiten Außenseiten der Gehäuse der beiden Kondensatoren deckungsgleich aneinander anliegen.

Weiterhin ist gemäß einer Ausführungsform vorgesehen, dass jeder der baugleichen Kondensatoren ein Elektrolytkondensator ist. Gemäß weiteren Ausführungsformen ist jeder der baugleichen Kondensatoren ein Tantal-Elektrolytkondensator oder ein Aluminium-Elektrolytkondensator.

Schließlich betrifft ein weiterer Aspekt der vorliegenden Erfindung ein Implantierbares medizinisches Gerät, aufweisend ein Gerätegehäuse, das einen Innenraum umgibt, wobei das Gerätegehäuse eine bogenförmige Innenseite aufweist, die durch eine bogenförmige lateralen Wandung des Gerätegehäuses gebildet ist, und wobei im Innenraum eine erfindungsgemäße Kondensatoranordnung angeordnet ist, wobei der zweite Abschnitt der lateralen Außenseite des Gehäuses des jeweiligen Kondensators sich entlang der Innenseite des Gerätegehäuses erstreckt und dieser insbesondere zugewandt ist.

Im Folgenden sollen Ausführungsformen der Erfindung sowie weitere Merkmale und Vorteile der Erfindung anhand der Figuren erläutert werden. Es zeigen:
- Fig. 1: eine perspektivische Ansicht einer Ausführungsform einer erfindungsgemäßen Kondensatoranordnung in Form eines Stapels aus drei erfindungsgemäßen Kondensatoren;
- Fig. 2: eine Draufsicht auf eine Ausführungsform eines erfindungsgemäßen Kondensators;

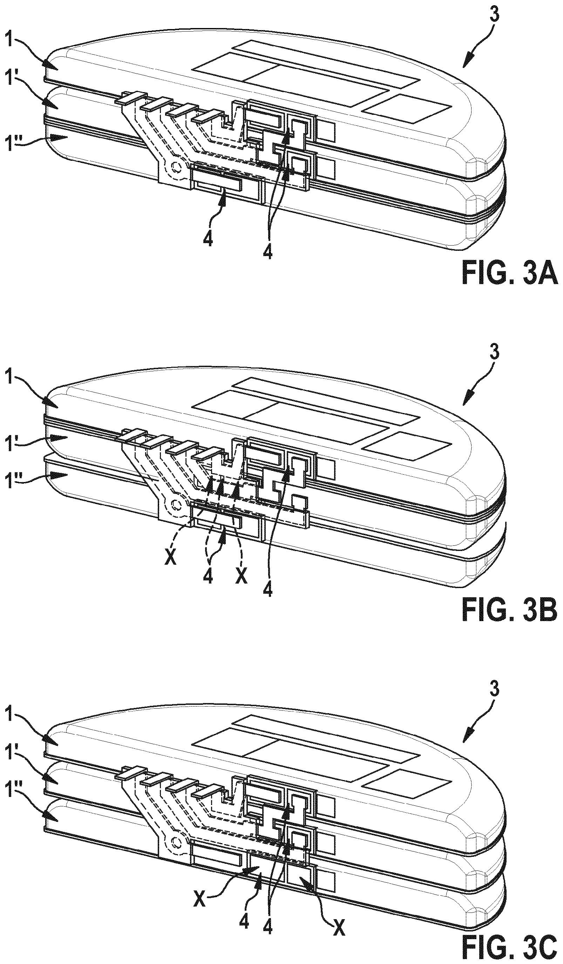

- Fig. 3: eine Ansicht dreier Stapel (A), (B), und (C) aus drei Kondensatoren nach Art der Figur 2, wobei die elektrischen Anschlüsse der Kondensatoren so angeordnet sind, dass eine verwechslungsfreie Montage der Kondensatoren unterstützt wird;
- Fig. 4: eine perspektivische Ansicht einer Ausführungsform eines erfindungsgemäßen implantierbaren medizinischen Gerätes;
- Fig. 5: eine perspektivische Ansicht der Kondensatoranordnung gemäß Figur 1, wobei die Figur 5 die Lage der Kondensatoranordnung in dem in der Figur 4 gezeigten Gerätegehäuse illustriert; und
- Fig. 6: eine schematische Darstellung einer Anordnung eines Kondensatorstapels mit bogenförmiger Außenseite (A) sowie eines quaderförmigen Kondensatorstapels (B) in einem Gerätegehäuse mit bogenförmiger Seitenwand;

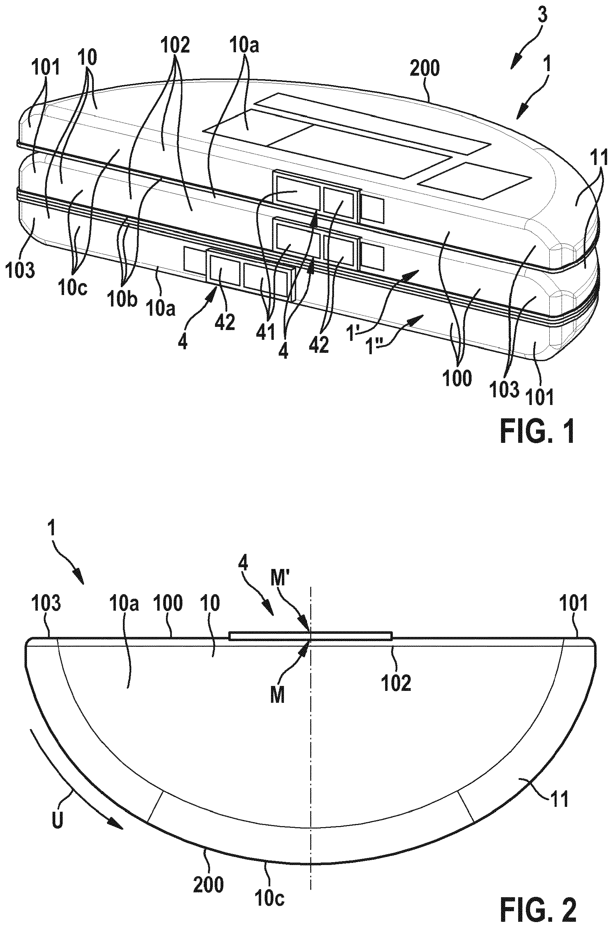

Figur 1 zeigt im Zusammenhang mit der Figur 2 eine Ausführungsform eines erfindungsgemäßen Kondensators 1, der für ein implantierbares medizinisches Gerät 2, z. B. in Form eines implantierbaren Kardioverter-Defibrillators (ICD), verwendbar ist.

Danach ist vorgesehen, dass der Kondensator 1 ein Gehäuse 10 aufweist, wobei das Gehäuse 10 eine erste Außenseite 10a sowie eine der ersten Außenseite 10a abgewandte zweite Außenseite 10b aufweist, wobei die beiden Außenseiten 10a, 10b über eine in einer Umfangsrichtung U des Gehäuses 10 umlaufende laterale Außenseite 10c des Gehäuses 10 miteinander verbunden sind, und wobei die laterale Außenseite 10c einen geraden ersten Abschnitt 100 sowie einen bogenförmigen zweiten Abschnitt 200 aufweist, wobei der erste Abschnitt 100 zwei Endbereiche 101, 103 aufweist, die über einen mittleren Bereich 102 des ersten Abschnitts 100 miteinander verbunden sind, wobei die Endbereiche 101, 103 jeweils mit dem zweiten Abschnitts 200 verbunden sind, und wobei der Kondensator 1 einen auf dem mittleren Bereich 102 des ersten Abschnitts 100 der lateralen Außenseite 10c angeordneten elektrischen Anschluss 4 aufweist, der zwei Kontaktflächen 41, 42 zum elektrischen Kontaktieren des Kondensators 1 aufweist.

Vorzugsweise ist das Gehäuse 10 des Kondensators 1 spiegelsymmetrisch zu einer Symmetrieebene E ausgebildet, die gemäß Figur 2 senkrecht zum ersten Abschnitt 100 der lateralen Außenseite 10c sowie senkrecht zu der ersten und der zweiten Außenseite 10a, 10b verläuft. Dabei ist vorzugsweise vorgesehen, dass die beiden Außenseiten 10a, 10b halbkreisförmig ausgebildet sind. Weiterhin ist vorzugsweise vorgesehen, wie insbesondere anhand der Figur 1 ersichtlich ist, dass der bogenförmige zweite Abschnitt 200 der lateralen Außenseite 10c mit der ersten Außenseite 10a eine bogenförmige, abgerundete Gehäusekante 11 des Gehäuses 10 des Kondensators 1 ausbildet.

Durch Drehung des Kondensators 1 um 180° ist dieser sowohl als oberer als auch als unterer Kondensator 1, 1" im Stapel verwendbar. Dies wird durch das symmetrische Design der Kondensatoraußenkontur ermöglicht, damit diese nach dem Drehen deckungsgleich mit den nicht gedrehten Kondensatoren ist.

Durch die Anordnung des elektrischen Anschlusses 4 des Kondensators 1 kommen die Anschlüsse 4 im Stapel nahe beieinander zu liegen, so dass die Weiterverdrahtung kompakt erfolgen kann. Wäre der jeweilige Anschluss 4 z. B. auf einem Endbereich 101, 103 des erste Abschnitts 100 der lateralen Außenseite 10c angeordnet, würde sich der Anschluss 4 nach einer 180°-Drehung des Kondensators 1 am entgegengesetzten Ende des Kondensatorstapels befinden.

Durch eine leicht außermittige Positionierung der Anschlüsse 4 wird weiterhin erreicht, dass die Anschlüsse 4 im Stapel zwar dicht genug beisammen positioniert sind, um sehr kurze Leiterbahnwege zu erreichen (verlustarme Verbindung durch geringen elektr. Widerstand), aber dennoch eine verwechslungssichere Anordnung realisierbar ist. Eine verwechslungsfreie Verbindung zum Verdrahtungsband ist notwendig, damit die richtige elektrische Polung eingehalten werden kann. Die besagte außermittige Anordnung des jeweiligen Anschlusses 4 wird gemäß Fig. 1 realisiert, indem der Mittelpunkt M' des elektrische Anschlusses 4 des jeweiligen Kondensators in der Umfangsrichtung U leicht versetzt zum Mittelpunkt M des ersten Abschnitts 100 angeordnet wird.

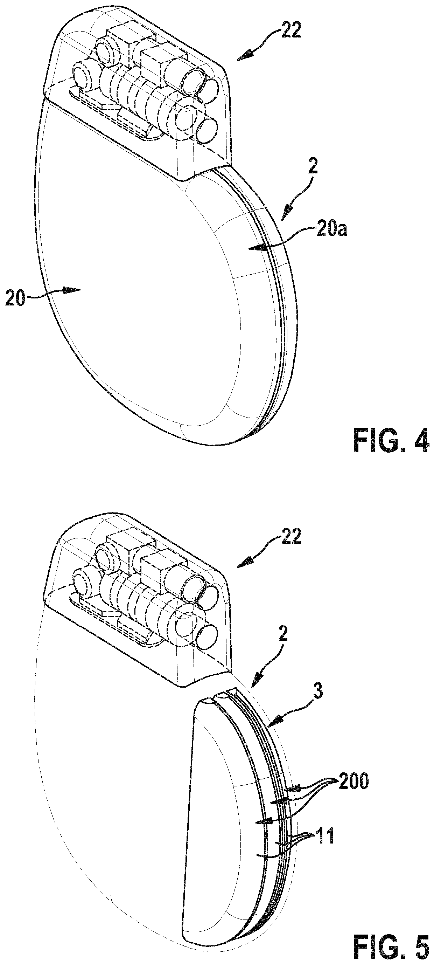

Der gewünschte Normalzustand, d.h., eine korrekt verdrahtete Kondensatoranordnung in Form eines Kondensatorstapels ist in der Figur 5(A) dargestellt. Figur 5(B) zeigt hingegen eine Situation in der der mittlere Kondensator 1' verdreht angeordnet ist oder der komplette Stapel um 180° gedreht ist. Hier ist aufgrund der besonderen Positionierung des jeweiligen Anschlusses 4 kein Schweißen der Anschlüsse 4 möglich. Schließlich zeigt Fig. 5(C) eine Situation, bei der keiner der Kondensatoren 1, 1', 1" gedreht angeordnet ist. Auch hier ist eine Verdrahtung nicht möglich. Weiterhin erlaubt die Fehlanordnung gemäß Fig. 5(C) auch keine Montage des Stapels 3 in dem Gerätegehäuse 20 des Implantats 2, da die eckige Stapelunterseite 10b nicht in das verrundete Gerätegehäuse 20 passt.

Die Figuren 4 bis 6 illustrieren schließlich die Anordnung der Kondensatoren 1, 1', 1" bzw. der Kondensatoranordnung im Innenraum 21 des Gerätegehäuses 20 des in der Figur 4 gezeigten implantierbaren medizinischen Gerätes 2, bei dem es sich hier um einen ICD 2 handelt, der des Weiteren über einen Header 22 verfügt, über den nicht gezeigte Elektroden des Gerätes 2 elektrisch leitend mit im Innenraum 21 des Gerätegehäuses 20 angeordneten Komponenten des Gerätes 2 verbindbar sind.

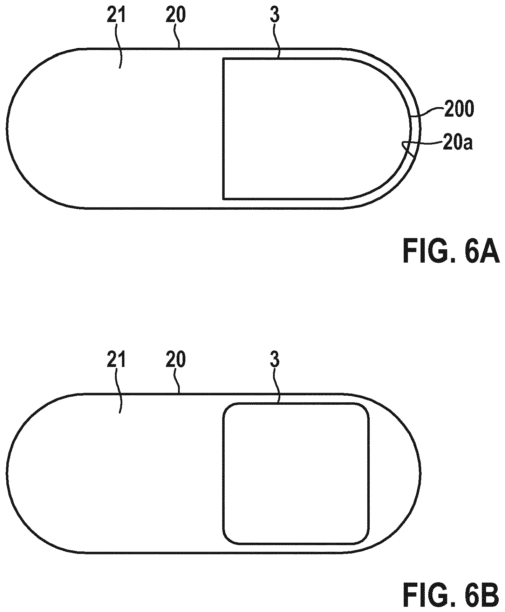

Figur 5 zeigt dabei den Kondensatorstapel 3 ohne das Gerätegehäuse 20, wobei ersichtlich wird, dass der bogenförmig erstreckte zweite Abschnitt 200 der lateralen Seite 10c des jeweiligen Kondensators einer bogenförmigen Innenseite des Gerätegehäuses folgt, so dass eine platzsparende Anordnung der Kondensatoranordnung im Innenraum 21 des Gerätegehäuses 20 erzielt wird. Diese Anordnung ist schematisch auch in der Figur 6(A) gezeigt. Die Figur 6(B) zeigt im Gegensatz hierzu die weniger vorteilhafte Anordnung eines quaderförmigen Kondensatorstapels 3' in einem Gerätegehäuse 21 mit bogenförmiger Innenseite bzw. Seitenwand 21a.

Die erfindungsgemäße Lösung erlaubt mit Vorteil eine volumeneffiziente Anordnung der Kondensatoren 1, 1', 1" im Gerätegehäuse 20 des Implantats 2. Vorzugsweise liegt der Verrundungsradius der besagten Kante 11 oberhalb von 2 mm. Durch die Erfindung wird weiterhin eine Reduzierung der Teilevielfalt und der Entwicklungskosten erreicht, da nur eine Kondensatorvariante im Stapel 3 verwendet wird. Durch die Ausgestaltung der Anschlüsse 4 ist eine verwechslungsfreie Montage möglich.

## Patentansprüche

1. Kondensatoranordnung für ein implantierbares medizinisches Gerät (2) aufweisend drei baugleiche Kondensatoren (1, 1', 1"), wobei jeder der baugleichen Kondensatoren (1, 1', 1") ein Gehäuse (10) umfasst, wobei das Gehäuse (10) eine erste Außenseite (10a) sowie eine der ersten Außenseite abgewandte zweite Außenseite (10b) aufweist, wobei die beiden Außenseiten (10a, 10b) über eine in einer Umfangsrichtung (U) des Gehäuses (10) umlaufende laterale Außenseite (10c) des Gehäuses (10) miteinander verbunden sind, und wobei die laterale Außenseite (10c) einen geraden ersten Abschnitt (100) sowie einen bogenförmigen zweiten Abschnitt (200) aufweist, wobei der erste Abschnitt (100) zwei Endbereiche (101, 103) aufweist, die über einen mittleren Bereich (102) des ersten Abschnitts (100) miteinander verbunden sind, wobei die Endbereiche (101, 103) jeweils mit dem zweiten Abschnitt (200) verbunden sind, und wobei der Kondensator (1) einen auf dem mittleren Bereich (102) des ersten Abschnitts (100) der lateralen Außenseite (10c) angeordneten elektrischen Anschluss (4) aufweist, der zwei Kontaktflächen (41, 42) zum elektrischen Kontaktieren des Kondensators (1) aufweist, und
der zweite Abschnitt (200) der lateralen Außenseite (10c) mit der ersten Außenseite (10a) eine abgerundete Gehäusekante (11) des Gehäuses (10) des Kondensators (1) ausbildet, und
die drei baugleichen Kondensatoren (1, 1', 1") aufeinander gestapelt angeordnet sind, so dass der jeweilige Kondensator (1, 1', 1") mit seiner ersten oder mit seiner zweiten Außenseite (10am 10b) an der ersten oder der zweiten Außenseite (10a, 10b) eines benachbarten Kondensators der Kondensatoranordnung (3) anliegt **dadurch gekennzeichnet, dass**
die drei baugleichen Kondensatoren (1, 1', 1") einen ersten äußeren Kondensator (1), einen zweiten mittleren Kondensator (1') und einen dritten äußeren Kondensator (1") umfassen, wobei der zweite Kondensator (1') zwischen den beiden äußeren Kondensatoren (1, 1") angeordnet ist, und wobei der zweite Kondensator (1') mit seiner zweiten Seite (10b) an der zweiten Seite (10b) des dritten Kondensators (1") anliegt, und wobei der zweite Kondensator (1') mit seiner ersten Seite (10a) an einer zweiten Seite (10b) des ersten Kondensators (1) anliegt, und
der elektrische Anschluss (4) des dritten Kondensators (1") versetzt zu den elektrischen Anschlüssen (4) des ersten und des zweiten Kondensators (1, 1') der Kondensatoranordnung (3) angeordnet ist.

2. Kondensatoranordnung nach Anspruch 1, **dadurch gekennzeichnet, dass** das Gehäuse (10) der baugleichen Kondensatoren (1, 1', 1") spiegelsymmetrisch zu einer Symmetrieebene (E) ausgebildet ist, die senkrecht zum ersten Abschnitt (100) der lateralen Außenseite (10c) sowie senkrecht zu der ersten und der zweiten Außenseite (10a, 10b) verläuft.

3. Kondensatoranordnung nach einem der vorhergehenden Ansprüche, **dadurch gekennzeichnet, dass** die erste und/oder die zweite Außenseite (10a, 10b) halbkreisförmig ausgebildet ist.

4. Kondensatoranordnung nach einem der vorhergehenden Ansprüche, **dadurch gekennzeichnet, dass** der erste Abschnitt (100) der lateralen Außenseite (10c) in der Umfangsrichtung (U) einen Mittelpunkt (M) aufweist, und dass der elektrische Anschluss (4) in der Umfangsrichtung einen Mittelpunkt (M') aufweist, wobei der Mittelpunkt (M') des elektrischen Anschlusses (4) in der Umfangsrichtung (U) zum Mittelpunkt (M) des ersten Abschnitts (100) versetzt angeordnet ist.

5. Kondensatoranordnung nach einem der vorhergehenden Ansprüche, **dadurch gekennzeichnet, dass** jeder der baugleichen Kondensatoren (1, 1', 1") ein Elektrolytkondensator ist.

6. Implantierbares medizinisches Gerät (2), aufweisend ein Gerätegehäuse (20), das einen Innenraum (21) umgibt, wobei das Gerätegehäuse (20) eine bogenförmige Innenseite (20a) aufweist, und wobei im Innenraum (21) eine Kondensatoranordnung (3) nach einem der Ansprüche 1 bis 5 angeordnet ist, wobei der zweite Abschnitt (200) der lateralen Außenseite (10c) des Gehäuses (10) des jeweiligen Kondensators (1, 1', 1") sich entlang der Innenseite (20a) des Gerätegehäuses (20) erstreckt.

## Claims

1. A capacitor assembly for an implantable medical device (2), having three structurally identical capacitors (1, 1', 1"), wherein each of the structurally identical capacitors (1, 1', 1") comprises a housing (10), wherein the housing (10) has a first outer side (10a) and a second outer side (10b) facing away from the first outer side, wherein the two outer sides (10a, 10b) are connected to one another via a lateral outer side (10c) of the housing (10) running in the circumferential direction (U) of the housing (10), and wherein the lateral outer side (10c) has a straight first portion (100) and an arcuate second portion (200), wherein the first portion (100) has two end regions (101, 103) which are connected to one another via a middle region (102) of the first portion (100), wherein the end regions (101, 103) are each connected to the second portion (200), and wherein the capacitor (1) has an electrical terminal (4) disposed on the middle region (102) of the first portion (100) of the lateral outer side (10c), said electrical terminal having two contact surfaces (41, 42) for electrically contacting the capacitor (1), and
the second portion (200) of the lateral outer side (10c) with the first outer side (10a) forms a rounded housing edge (11) of the housing (10) of the capacitor (1), and
the three structurally identical capacitors (1, 1', 1") are arranged stacked on top of one another so that each capacitor (1, 1', 1") rests with its first or with its second outer side (10a, 10b) against the first or the second outer side (10a, 10b) of an adjacent capacitor of the capacitor assembly (3),
**characterised in that**
the three structurally identical capacitors (1, 1', 1") comprise a first outer capacitor (1), a second middle capacitor (1') and a third outer capacitor (1"), wherein the second capacitor (1') is disposed between the two outer capacitors (1, 1"), and wherein the second capacitor (1') rests with its second side (10b) against the second side (10b) of the third capacitor (1"), and wherein the second capacitor (1') rests with its first side (10a) against a second side (10b) of the first capacitor (1), and
the electrical terminal (4) of the third capacitor (1") is disposed offset relative to the electrical terminals (4) of the first and the second capacitor (1, 1') of the capacitor assembly (3).

2. The capacitor assembly according to claim 1, **characterised in that** the housing (10) of the structurally identical capacitors (1, 1', 1") is mirror-symmetrical to a plane of symmetry (E) that runs perpendicular to the first portion (100) of the lateral outer side (10c) and perpendicular to the first and the second outer side (10a, 10b).

3. The capacitor assembly according to either one of the preceding claims, **characterised in that** the first and/or the second outer side (10a, 10b) is semi-circular.

4. The capacitor assembly according to any one of the preceding claims, **characterised in that** the first portion (100) of the lateral outer side (10c) has a centre point (M) in the circumferential direction (U), and **in that** the electrical terminal (4) has a centre point (M') in the circumferential direction, wherein the centre point (M') of the electrical terminal (4) in the circumferential direction (U) is disposed offset relative to the centre point (M) of the first portion (100).

5. The capacitor assembly according to any one of the preceding claims, **characterised in that** each of the structurally identical capacitors (1, 1', 1") is an electrolytic capacitor.

6. An implantable medical device (2), having a device housing (20) which surrounds an interior (21), wherein the device housing (20) has an arcuate inner side (20a), and wherein a capacitor assembly (3) according to any one of claims 1 to 5 is disposed in the interior (21), wherein the second portion (200) of the lateral outer side (10c) of the housing (10) of the respective capacitor (1, 1', 1") extends along the inner side (20a) of the device housing (20).

## Revendications

1. Ensemble de condensateurs pour dispositif médical implantable (2) présentant trois condensateurs (1, 1', 1") de construction semblable, dans lequel chacun des condensateurs (1, 1', 1") de construction semblable présente un boitier (10), où le boitier (10) présente une première face extérieure (10a) ainsi qu'une deuxième face extérieure (10b), opposée à la première face extérieure, où les deux faces extérieures (10a, 10b) sont reliées ensemble par le biais d'une face extérieure latérale périphérique (10c) du boitier (10) dans une direction périphérique (U) du boitier (10), et où la face extérieure latérale (10c) présente un premier segment linéaire (100) ainsi qu'un deuxième segment (200) en forme d'arc, où le premier segment (100) présente deux zones d'extrémités (101, 103) qui sont reliées ensemble par une zone centrale (102) du premier segment (100), où les zones d'extrémité (101, 103) sont respectivement reliées avec le deuxième segment (200), et où le condensateur (1) présente une connexion électrique (4) disposée sur la zone centrale (102) du premier segment (100) de la face extérieure latérale (10c), qui présente deux surfaces de contact (41, 42) pour le contact électrique du condensateur (1), et
le deuxième segment (200) de la face extérieure latérale (10c) forme avec la première face extérieure (10a) une arête de boitier (11) arrondie du boitier (10) du condensateur (1), et
les trois condensateurs (1, 1', 1") de construction semblable sont disposés empilés les uns sur les autres de sorte que le condensateur (1, 1', 1") respectif est adjacent à la première ou à la deuxième face extérieure (10a, 10b) d'un condensateur voisin de l'ensemble de condensateurs (3) avec sa première ou avec sa deuxième face extérieure (10a, 10b),
**caractérisé en ce que**
les trois condensateurs (1, 1', 1") de construction semblable comprennent un premier condensateur extérieur (1), un deuxième condensateur central (1') et un troisième condensateur extérieur (1"), où le deuxième condensateur (1') est disposé entre les deux condensateurs extérieurs (1, 1"), et où le deuxième condensateur (1') est adjacent avec sa deuxième face (10b) à la deuxième face (10b) du troisième condensateur (1"), et où le deuxième condensateur (1') est adjacent avec sa première face (10a) à une deuxième face (10b) du premier condensateur (1), et
la connexion électrique (4) du troisième condensateur (1") est décalée par rapport aux connexions électriques (4) des premier et deuxième condensateurs (1, 1') de l'ensemble de condensateurs (3).

2. Ensemble de condensateurs selon la revendication 1, **caractérisé en ce que** le boitier (10) des condensateurs (1, 1', 1") de construction semblable est conçu symétrique en miroir par rapport à un plan de symétrie (E) qui s'étend perpendiculairement par rapport au premier segment (100) de la face extérieure latérale (10c) ainsi que perpendiculairement par rapport aux première et deuxième faces latérales (10a, 10b).

3. Ensemble de condensateurs selon l'une des revendications précédentes, **caractérisé en ce que** les première et/ou deuxième faces latérales (10a, 10b) sont conçues en forme de demis cercles.

4. Ensemble de condensateurs selon l'une des revendications précédentes, **caractérisé en ce que** le premier segment (100) de la face extérieure latérale (10c) présente un point central (M) dans la direction périphérique (U) et que la connexion électrique (4) présente un point central (M') dans la direction périphérique, où le point central (M') de la connexion électrique (4) est disposé décalé dans la direction périphérique (U) par rapport au point central (M) du premier segment (100).

5. Ensemble de condensateurs selon l'une des revendications précédentes, **caractérisé en ce que** chacun des condensateurs (1, 1', 1") de construction semblable est un condensateur électrolytique.

6. Dispositif médical implantable (2) présentant un boitier de dispositif (20) qui entoure un espace intérieur (21), dans lequel le boitier de dispositif (20) présente une face intérieure (20a) en forme d'arc de cercle et dans lequel un ensemble de condensateurs (3) selon l'une des revendications 1 à 5 est disposé dans l'espace intérieur (21), dans lequel le deuxième segment (200) de la face extérieure latérale (10c) du boitier (10) du condensateur (1, 1', 1") respectif s'étend le long de la face intérieure (20a) du boitier de condensateur (20).
